# EUROPÄISCHE PATENTSCHRIFT

(11) **EP 0 520 262 B1**
(45) Veröffentlichungstag und Bekanntmachung des Hinweises auf die Patenterteilung: **28.08.1996**
(21) Anmeldenummer: 92109973.5
(22) Anmeldetag: 13.06.1992
(51) Int. Cl.: G01N 33/52, G01N 21/64

(54) **Chemischer Sensor**
Chemical sensor
Capteur chimique

(30) Priorität: 28.06.1991 DE 4121426
(43) Veröffentlichungstag der Anmeldung: 30.12.1992
(73) Patentinhaber: BASF Aktiengesellschaft, 67063 Ludwigshafen (DE)
(72) Erfinder: Schrepp, Wolfgang, Dr., W-6900 Heidelberg (DE); Ramesh, Chandra Ahuja, Dr., W-3400 Göttingen (DE); Budach, Wolfgang, W-3400 Göttingen (DE); Moebius, Dietmar, Dr., W-3401 Waake (DE)

(56) Entgegenhaltungen:
- EP-A- 0 429 907
- BERICHTE DER BUNSENGESELLSCHAFT FÜR PHYSIKALISCHE CHEMIE, Band 82, Nr. 9, September 1978, Weinheim (DE); D.M. BIUS, Seiten 848-858
- THE ANALYST, Band 115, Nr. 4, April 1990, London (GB); J.N. ROE et al., Seiten 353-358
- THE ANALYST, Band 113, Nr. 5, Mai 1988, London (GB); B.P.H. SCHAFFAR et al., Seiten 693-697
- THIN SOLID FILMS, Band 160, 1988, Lausanne (CH); M. AIZAWA et al., Seiten 477-481
- NATURE, Band 320, Nr. 6058, 13 März 1986, London (GB); T.H. WATTS et al., Seiten 179-181
- CHEMISTRY LETTERS, Nr. 2, 1989, Tokyo (JP); S. YOSHIDA et al., Seiten 243-246

## Beschreibung

Die Erfindung betrifft einen chemischen Sensor, der aus einem festen Trägermaterial und darauf aufgebrachten diskreten Schichten unterschiedlicher Zusammensetzung besteht und sich zum Nachweis von Ionen oder organischen Molekülen eignet.

Sensoren auf der Basis von Fluoreszenzfarbstoffen sind schon seit längerem bekannt. So beschreiben M. Azzawa et al. (Thin Solid Films, 160 (1988) 477) einen Sensor, bei dem eine oder mehrere Schichten eines Fluoreszenzfarbstoffes mit dem zu untersuchenden Analyten in Kontakt gebracht wird. Durch Wechselwirkung mit einem oder mehreren Bestandteilen des Analyten tritt eine Änderung der Fluoreszenzintensität ein. In der Regel werden fluoreszierende Stoffe verwendet, bei denen eine Verringerung der Fluoreszenz, ein Quenchen, eintritt. Eine ähnliche Anwendung auf dem Gebiet der Biosensorik wird durch M. Azzawa et al. in Thin Solid Films, 180 (1989) 227 beschrieben.

Weiterhin sind Sensoren bekannt, die auf einem Fluoreszenztransfer beruhen, bei dem die Fluoreszenz des Farbstoffes 1 auf einen zweiten Fluoreszenzfarbstoff übertragen wird. Diese Systeme benutzen einen zweiten Fluoreszenzfarbstoff, der z.B. an einen Bestandteil des Analyten gebunden sein kann (s. z.B. EP 150 905 oder EP 429 907). Auch ein Nachweis des Energietransfers in Wellenleiterstrukturen mittels evaneszenter Wellen ist beschrieben (Nature 320 (1986) 179).

Von O.S. Wolfbeis et al. sind eine Reihe von Sensoren beschrieben, die ein Quenchen der Fluoreszenz aufgrund Potential empfindlicher Farbstoffe nutzen, wobei teilweise die Langmuir-Blodgett-Technik eingesetzt wird (Analyst, 113 (1988) 693; Anal. Chim. Acta, 217 (1989) 1).

Von J.N. Roe et al. (Analyst, 115 (1990) 353) wird ein faseroptischer Sensor beschrieben, bei dem Fluorophor und das auf Kalium-Ionen empfindliche Ionophor Valinomycin in einer ca. 6 µm dicken, inhomogenen Polymerschicht auf die Faser aufgebracht werden.

Die bisher beschriebenen Sensoren beruhen alle entweder auf einem direkten Quenchen der Fluoreszenz durch den Analyten, befinden sich zusammen mit einem Absorber in einer Polymermatrix oder verwenden potential-empfindliche Farbstoffe.

Die Responsezeiten solcher Sensoren sind durch die Diffusion des Analyten im Sensorsystem limitiert und es findet meist eine Referenzelektrode Verwendung, die die Selektivität erhöhen soll. Vielfach werden dazu sogenannte "Dip Coat"-Schichten benutzt, die relativ dick sind.

Aufgabe der vorliegenden Erfindung ist es, einen chemischen Sensor aufzuzeigen, der wesentlich kürzere Responsezeiten aufweist, reproduzierbar und relativ einfach herzustellen ist und noch bei sehr hohen Verdünnungen des Analyten (10⁻¹⁰ bis 10⁻⁶-molar) einsetzbar ist.

Diese Aufgabe konnte überraschenderweise mit dem erfindungsgemäßen chemischen Sensor gelöst werden. Gegenstand der vorliegenden Erfindung ist ein chemischer Sensor, bestehend aus
(a) einem festen Trägermaterial,
(b) einer auf dem Trägermaterial aufgebrachten Schicht (D), die mindestens eine fluoreszierende Verbindung enthält, und
(c) einer auf der Schicht (D) aufgebrachten Schicht (A), die eine oder mehrere Kupplungskomponenten (K) enthält, die durch Reaktion mit dem Analyten eine oder mehrere spezifische Absorptionsbanden hervorrufen, die mit der Emission der fluoreszierenden Verbindung der Schicht (D) teilweise oder vollständig spektral überlappen.

Eine mögliche weitere Ausgestaltung des erfindungsgemäßen chemischen Sensors besteht darin, daß zwischen Schicht (D) und Schicht (A) eine Zwischenschicht (Z) mit definiertem Dipolmoment zur Kontrolle der chemischen Gleichgewichte in den benachbarten Schichten angebracht ist, derartige Zwischenschichten (Z) können beispielsweise aus langkettigen Alkoholen, Carbonsäuren, Carbonsäureestern, Aminen oder inerten organischen Polymeren bestehen. Die auf dem festen Trägermaterial aufgebrachten Schichten weisen vorzugsweise insgesamt eine Dicke zwischen 20 und 200 Å auf.

Bevorzugte Ausführungsformen des erfindungsgemäßen chemischen Sensors bestehen auch darin, daß das feste Trägermaterial aus Quarz oder Glas besteht und gegebenenfalls vorbehandelt ist, sowie daß die Kupplungskomponenten (K) der Schicht (A) an der Oberfläche des Sensors in direktem Kontakt mit der Lösung des Analyten sind.

Der Abstand zwischen fluoreszierender Verbindung der Donorschicht (D) und der Kupplungskomponenten der Akzeptorschicht (A) entspricht vorzugsweise dem Försterradius.

Die Donorschicht (D) kann entweder aus der fluoreszierenden Verbindung bestehen oder die fluoreszierende Verbindung kann in einer Polymer- oder Monomermatrix verteilt oder gebunden sein.

Bevorzugt ist ferner, daß in der Schicht (D) J- oder Scheibe-Aggregate ausgebildet sind.

Die Kupplungskomponente (K) der Akzeptorschicht (A) kann eine Verbindung sein, die mit Ionen oder organischen Molekülen im Sinne der Wirts/Gast-Reaktion wechselzuwirken vermag, z.B. Einschlußverbindungen bei Clathraten oder Kryptanden, oder die Kupplungskomponenten (K) können aus einem oder mehreren Komplexbildnern bestehen, die mit Ionen, insbesondere Schwermetallionen zu reagieren vermögen.

Die Schichten (D) und (A) sowie gegebenenfalls (Z) können nach verschiedenen Techniken auf das feste Trägermaterial aufgebracht werden, beispielsweise nach dem Schleuderverfahren (spin coating), durch Adsorption aus Lösung, nach dem Aufdampfverfahren, dem Self-Assembley-Verfahren sowie vorzugsweise nach der Langmuir-Blodgett- und/oder Langmuir-Schäfer-Technik.

Beim erfindungsgemäßen chemischen Sensor befindet sich also auf einem geeigneten Substrat (= festes Trägermaterial) eine Donorschicht (D), die einen oder mehrere geeignete Fluoreszenzfarbstoffe enthält. In einer darüberliegenden Akzeptorschicht (A) befindet sich eine Kupplungskomponente (K), die in der Lage ist, mit dem zu untersuchenden Analyten oder einem Bestandteil desselben eine Reaktion einzugehen. Durch diese "Reaktion" wird ein Akzeptor gebildet, der mit der Emission des Fluoreszenzfarbstoffs in der Donorschicht (D) spektral überlappt. Vor der Reaktion ist die Absorption der Kupplungskomponente (K) relativ gering und im Bereich der Emission von (D). Durch Reaktion mit dem Analyten entsteht eine Verbindung, die ein geändertes Absorptionsspektrum aufweist, das damit eine Änderung der Überlappung mit dem Donorfluoreszenzspektrum bewirkt. Hierdurch wird ein Quenchen der Donor-Fluoreszenz bewirkt, das leicht nachweisbar ist.

Bevorzugt erfolgt die Herstellung der Donorschicht (D) und der Schicht (A), welche die Kupplungskomponente (K) enthält und die nach Wechselwirkung mit dem Analyten zur Akzeptorschicht (A) wird, mit Hilfe der Langmuir-Blodgett oder Langmuir-Schäfer-Technik, deren Prinzip z.B. in W.J. Feast und H.S. Munro, Polymer Surfaces and Interfaces, J. Wiley & Sons, Chichester 1987, S. 163ff beschrieben ist, oder durch Chemisorption nach der Self-Assembly Technik (vgl. Thin Solid Films, 132 (1985) 135). Wie dem Fachmann bekannt, erlauben die genannten Verfahren die 5 Herstellung sehr definierter und geordneter Schichten. Schichtabstände sind bis hinunter in den AE-Bereich einstellbar.

Gegenüber den bisher beschriebenen Sensoren erlaubt der erfindungsgemäße chemische Sensor die definierte Nutzung des sogenannten Förster-Transfers (vgl. Th. Förster, Fluoreszenz Organischer Verbindungen, Vandenhoeck & Ruprecht, Göttingen, (1950) Seiten 67 - 86; H. Kuhn et al. in Weissberger und Rossiter (Hrsg.), Physical Methods of Chemistry, Teil III B, Wiley, New York 1972, S. 577ff). Von Förster-Transfer spricht man, wenn die Fluoreszenzenergie der Donorschicht auf die Akzeptorschicht strahlungslos bzw. resonant übertragen wird. Dieser Energietranser ist sehr stark vom Abstand zwischen Donor und Akzeptor abhängig. Die Effektivität des Energietransfers wird nach Förster durch einen kritischen Abstand zwischen Donor und Akzeptor, dₒ, beschrieben. Dieser Abstand dₒ beträgt in der Regel zwischen 20 und 100 AE. Wie in der oben zitierten Arbeit von H. Kuhn et al. nachgewiesen, ist die Langmuir-Blodgett-Technik zur Herstellung molekularer Überstrukturen (Assemblies), die nach dem Förster-Prinzip arbeiten, sehr gut geeignet.

Durch das Prinzip des Förster-Transfers kann eine hohe Empfindlichkeitssteigerung und Selektivität erreicht werden. Dies wird unmittelbar deutlich, wenn wir als Beispiel monomolekulare Schichten betrachten. Im allgemeinen absorbiert eine monomolekulare Farbstoffschicht im Bereich des Absorptionsmaximums einige Prozent des einfallenden Lichtes. Das Fluoreszenzlicht einer Donorschicht wird demnach von einer über 100AE entfernten Akzeptorschicht nur zu einem geringen Bruchteil absorbiert. Unterhalb eines bestimmten Abstands kommt es jedoch zum Förster Energietransfer: Sind Donor- und Akzeptorschicht beispielsweise um den sogenannten Försterradius dₒ (Th. Förster, Fluoreszenz, Organischer Verbindungen, Vandenhoeck & Ruprecht, Göttingen, (1950) voneinander entfernt, so ist die Fluoreszenzintensität des Donors um ca. 50 % gelöscht. Die entsprechende Energie wird strahlungslos, bzw. resonant auf den Akzeptor übertragen und von diesem seinerseits durch Emission oder durch strahlungslose Desaktivierung abgegeben. Die Fluoreszenzintensität des Donors zeigt in Abhängigkeit vom Akzeptorabstand ein S-förmiges Verhalten, wobei der Wendepunkt dem Försterradius entspricht. Beträgt der Abstand weniger als 20 % des Försterradius, so ist die Donorfluoreszenz nahezu vollständig gelöscht. Der Försterradius liegt bei vielen Farbstoffen zwischen 20 und 100 AE. Im Falle der auf den Seiten 8 bis 10 zugegebenen Farbstoffe beträgt der Radius ca. 50 AE. Durch die Überlappung zwischen der charakteristischen Fluoreszenz eines Donorfarbstoffs mit der charakteristischen Absorption eines während der Messung in der benachbarten Schicht gebildeten Akzeptors (Förster-Energietransfer), sowie durch kontrollierten Schichtaufbau, wird eine hohe Selektivität und Empfindlichkeit des Sensors erzielt.

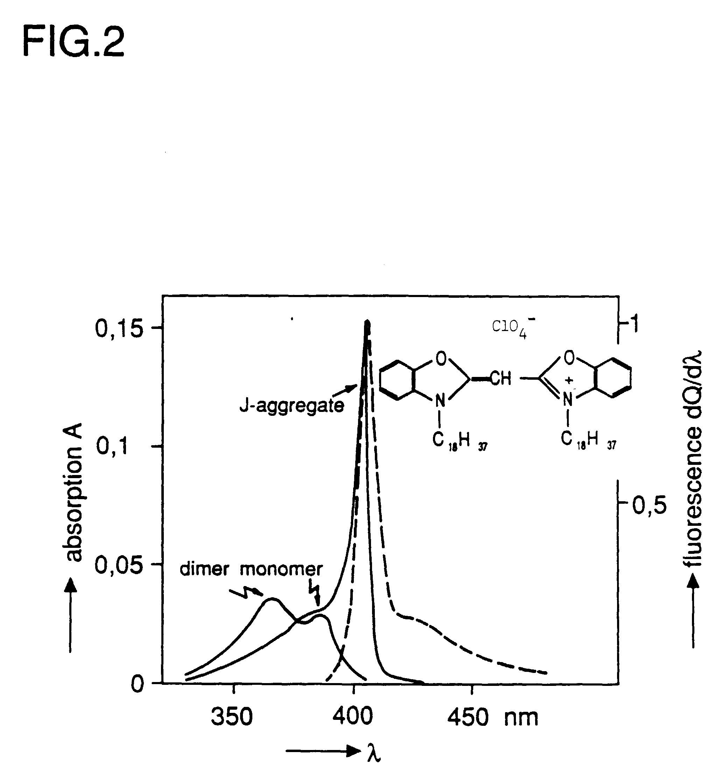

Eine weitere Steigerung der Effektivität des erfindungsgemäßen Sensorprinzips wird möglich, wenn statt normaler Fluoreszenzfarbstoffe Aggregate (z.B. J- oder Scheibe-) in der Schicht (D) benutzt werden. Scheibeaggregate (vgl. H. Kuhn et al.) resultieren aus einer speziellen Aggregation von Farbstoffen und sind mit Hilfe der Langmuir-Blodgett-Technik herstellbar. Sie zeichnen sich durch eine sehr schmalbandige Fluoreszenz aus und sind somit im Sinne des erfindungsgemäßen Sensorprinzips sehr effizient nutzbar. Sie können eine Empfindlichkeitssteigerung bewirken, da bei schmaler Fluoreszenzbande eine vollständige spektrale Überlappung mit einer Akzeptorbande leichter möglich ist. Außerdem kann so auch die Selektivität des Sensors erhöht werden, da die Bildung des Akzeptors aus Kupplungskomponente (K) und Analyt für verschiedene Analyten oder verschiedene Analytenbestandteile zu unterschiedlichen Spektren führen kann. Durch geeignete Wahl des Fluoreszenzfarbstoffes oder Scheibeaggregates kann hier eine Diskriminierung erreicht werden. Eine schematische Verdeutlichung ist in Figur 2 angegeben (aus D. Möbius, Ber. Bunsenges. Phys. Chem., 82 (1978) 848).

Da die funktionellen Einheiten des erfindungsgemäßen Sensors (Donormoleküle der Schicht (D) und Kupplungskomponenten (K)) in separate Schichten eingebaut sind, können Aggregation bzw. Orientierung, sowie die sonstigen Filmeigenschaften beeinflußt und den Anforderungen angepaßt werden.

Beim erfindungsgemäßen Sensor sind eine Reihe naheliegender Veränderungen denkbar, die das Prinzip aber nicht beeinflussen. So ist denkbar, daß Donor- und/oder Akzeptorschicht aus jeweils einer oder mehreren Schichten bestehen, daß mehrere Fluoreszenzfarbstoffe und/oder Kupplungskomponenten benutzt werden, daß Zwischenschichten aufgebracht sein können (z.B. um die Haftung zum Substrat zu verbessern oder zur Abstandseinstellung zwischen Donor- und Akzeptorschicht) sowie Zwischenschichten mit definiertem Dipolmoment zur Kontrolle der chemischen Gleichgewichte in den benachbarten Schichten.

Die obigen Ausführungen begründen eine Reihe von Vorteilen des erfindungsgemäßen Sensorprinzips. Der schichtweise Aufbau des Sensors erlaubt eine optimale Anpassung von Fluorophor und Kupplungskomponente an die nachzuweisende Spezies. Insbesondere bei der Nutzung der Langmuir-Blodgett-Technik kann der Sensor sehr definiert und reproduzierbar hergestellt werden. Durch Nutzung des Förster-Transfers, speziell bei der Verwendung von Scheibeaggregaten können Sensoren hoher Effektivität und Selektivität aufgebaut werden. Durch geeignete Kombination von Donor- und Akzeptorkomponenten können mehrere Spezies gleichzeitig nachgewiesen werden.

Gegenüber Sensoren, die Fluorophor und Absorberkomponente in ein und derselben Schicht enthalten, ergeben sich folgende Vorteile: Der für den Förster-Transfer entscheidende Abstand ist definiert und reproduzierbar herstellbar. Die spektroskopischen Vorteile von Scheibeaggregaten (auch J-Aggregate genannt) sind nutzbar. Es ist bekannt, daß sowohl Fluorophore als auch die Akzeptoren relativ undefinierte Aggregate oder Domänen bilden können, so daß beim Vorhandensein in einer Schicht nicht die volle Effektivität genutzt werden kann, da nur ein Teil der vorhandenen Systeme in definiertem Abstand vorliegt. Die Bandbreite möglicher Donoren und Akzeptoren wird beim Vorhandensein in ein und derselben Schicht eingeschränkt, da ein für beide Komponenten kompatibles Lösungsmittel und eine kompatible Matrix gefunden werden muß.

Der erfindungsgemäße Sensor benötigt lediglich 2 Hauptkomponenten (Donorfarbstoff und Kupplungskomponente), die in separate, benachbarte Schichten eingebaut werden. Entmischungsphänomene werden so vermieden. Die komplexierende hydrophile Kupplungskomponente (K) befindet sich im direkten Kontakt mit der wäßrigen Lösung des Analyten. Der so erhaltene Akzeptorkomplex wird an der Grenzfläche fest/flüssig gebildet. Die Geschwindigkeit des Sensors ist somit lediglich von der Art der chemischen Bindung und von der Geometrie des Reaktionsgefäßes abhängig. Eine Verzögerung der Sensorantwort aufgrund von Diffusion des Analyten durch das Sensorsystem entfällt.

Insgesamt ist also das durch den erfindungsgemäßen Sensor erreichte analytische Konzept flexibel, definiert sensitiv, selektiv, reproduzierbar, schnell und vom Prinzip her sehr allgemein anwendbar.

Zu den Aufbaukomponenten und zur Herstellung des erfindungsgemäßen Sensors ist im einzelnen folgendes auszuführen.

Für den Sensor aus einer auf einem festen Trägermaterial aufgebrachten Donorschicht (D) und einer Schicht (A), die eine Kupplungskomponente (K) enthält und die nach Reaktion mit einem Analyten zu einer Akzeptorschicht wird, können als festes Trägermaterial Verwendung finden: Glas, Quarz, Halbleitermaterialien wie Germanium, GaAs oder Silizium, Li-niobat, Zinkselenid, Porzellan, metallische Schichten, Lichtleiterfaser, Wellenleiterstrukturen, Kunststoffe sowie speziell vorbehandelte Substrate wie z.B. hydrophobisierte, plasmabehandelte oder vorbeschichtete Träger. Quarz und Glas, das gegebenenfalls hydrophobisiert sein kann, sind bevorzugt.

Die Schichtherstellung erfolgt bevorzugt mit Hilfe der Langmuir-Blodgett-Technik. Wie dem Fachmann bekannt, handelt es sich hierbei um ein Verfahren zur Übertragung monomolekularer Schichten auf feste Träger. Die monomolekulare Schicht wird dabei auf der Flüssigkeitsoberfläche (i.a. Wasser) eines Langmuir-Troges durch Kompression erzeugt, nachdem die Lösungen der auf den Träger aufzubringenden Substanzen auf der Wasseroberfläche gespreitet worden sind. In der Regel handelt es sich hierbei um amphiphile Substanzen. Üblicherweise erfolgt die Übertragung des Monofilms auf das meist speziell vorbehandelte Substrat durch Ein- und Austauchen durch den Film auf der Flüssigkeitsoberfläche unter Verwendung eines sogenannten Filmliftes. Das Substrat kann sowohl senkrecht zur Flüssigkeitsoberfläche als auch unter einem bestimmten Winkel getaucht werden (Langmuir-Blodgett-Technik) als auch parallel zur Flüssigkeitsoberfläche ("horizontal dipping"). Wird der Träger punktuell oder mit einer Kante mit der Flüssigkeitsoberfläche in Verbindung gebracht und dann auf die Oberfläche geklappt, spricht man von Langmuir-Schäfer-Technik.

Die Herstellung der Schichten kann aber auch nach anderen Verfahren erfolgen, wobei u.U. Eigenschaftseinbußen hingenommen werden müssen. So ist eine Schichtaufbringung z.B. auch über definierte Chemisorptionsprozesse (Self-Assembly) möglich. Hierbei werden zumeist langkettig modifizierte organische Verbindungen in mehr oder weniger polaren Lösungsmitteln in relativ verdünnter Form gelöst. Taucht man nun ein geeignet vorbehandeltes Substrat in diese Lösung ein, findet eine geordente Chemisorption am Träger statt. Die Schichten können hoch geordnet sein, Multischichten sind herstellbar. Weitere Verfahren wären z.B. Plasmapolymerisation, Schleudertechnik, Aufdampfverfahren und Physisorption.

Bei der Herstellung des Sensors können für die einzelnen Schichtsysteme auch unterschiedliche Herstellverfahren zur Anwendung kommen. So kann z.B. die Vorbehandlung des Trägers mittels Chemisorption erfolgen, gen, die Herstellung der aktiven Schichten (D) und (A) mittels Langmuir-Blodgett-Technik.

Beispiele für fluoreszierende Verbindungen in der Donorschicht (D) sind folgende Fluoreszenzfarbstoffe 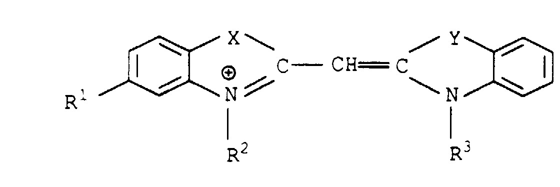 X, Y unabhängig voneinander für O, S, Se,
R¹, R², R³ unabhängig voneinander für C₁-C₂₂-Alkyl, 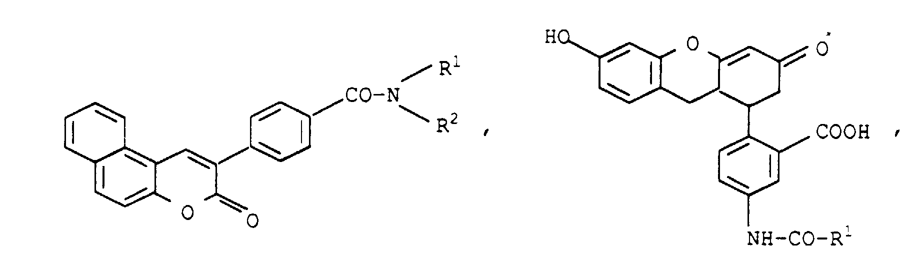 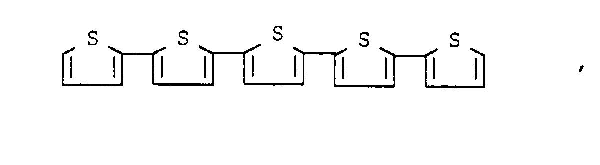 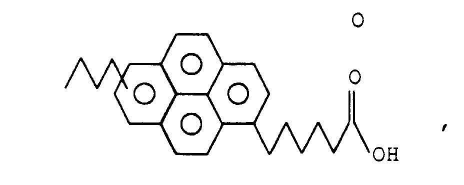 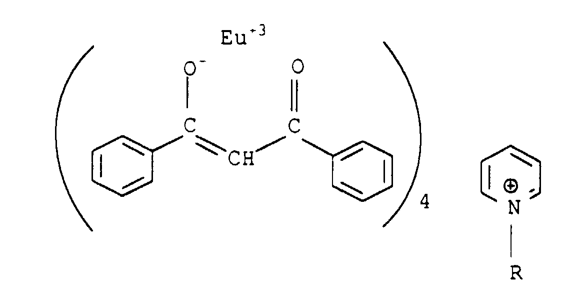 mit R = C₁₅- bis C₃₀-Alkyl

Eine weitere Auswahl geeigneter Farbstoffe findet sich z.B. in der bereits oben angeführten Publikation von H. Kuhn et al. Wird die Donorschicht mittels Langmuir-Blodgett-Technik hergestellt, so können die Fluoreszenzfarbstoffe unmodifiziert in eine Matrix aus für die Langmuir-Blodgett-Technik geeigneten Substanzen z.B. langkettige Fettsäuren oder modifizerte Polymere eingemischt werden. Die Farbstoffe können auch so modifiziert sein, daß sie direkt für die Langmuir-Blodgett-Technik geeignet sind, beispielsweise 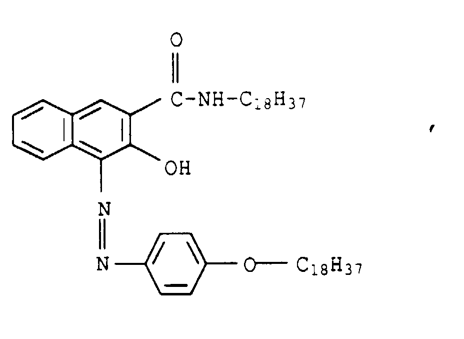 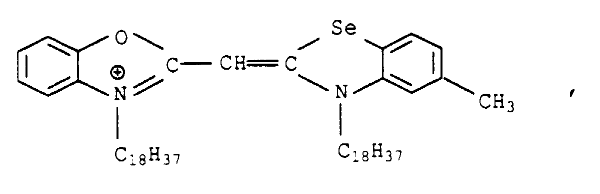 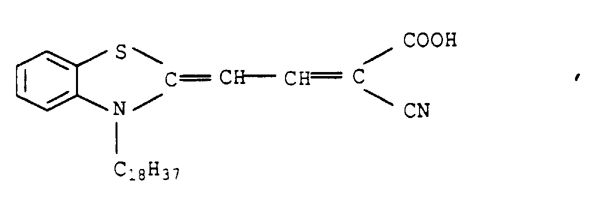 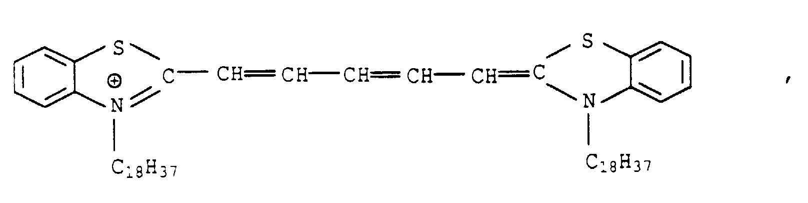 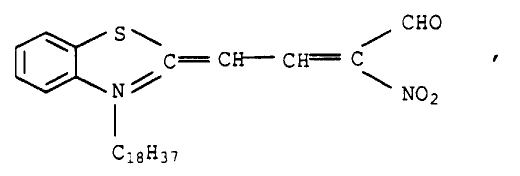 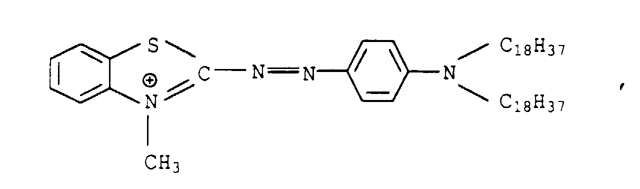 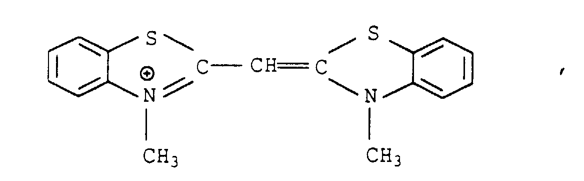 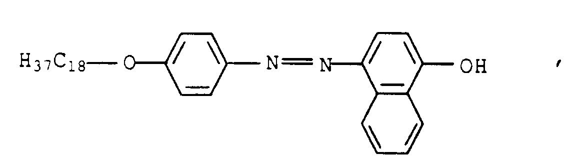 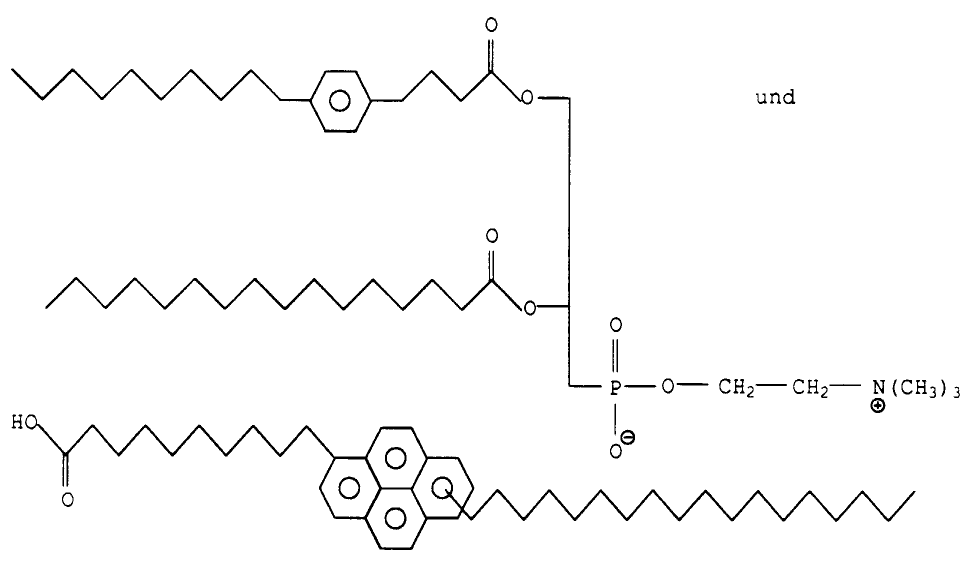 Eine direkte Anbindung der Fluoreszenzfarbstoffe an Polymere ist ebenfalls denkbar, auch die Anbindung an für die Langmuir-Blodgett-Technik geeignete (vgl. z.B. F. Embs et al., Adv. Mat., 3 (1991) 25). Weitere geeignete Polymere sind z.B. in DE-OS 38 30 325 oder DE-OS 38 30 862 beschrieben.

Wie oben schon erwähnt, kann die gezielte Aggregation von Fluoreszenzfarbstoffen zu einer deutlichen Erhöhung der Fluoreszenz führen und somit zu einer Empfindlichkeits- und Selektivitätssteigerung des Sensors führen. In Figur 2 ist ein zur Aggregatbildung geeigneter Farbstoff mit zugehörigem Spektrum gezeigt. Die Herstellung von J- oder Scheibe-Aggregaten kann auf der Wasseroberfläche eines Langmuir-Troges aus einer 1:1 Mischung von N',N-Dioctadecyl-oxacyaninperchlorat mit Hexadekan, in Chloroform gelöst, geschehen.

Erfindungsgemäß wird auf die Donorschicht (D) eine Schicht (A) mit einer oder mehreren Kupplungskomponenten (K) aufgebracht: Für die daraus erhältliche Akzeptorschicht ist entscheidend, daß eine Änderung des Absorptionsspektrums eintritt.

Geeignete Substanzen mit Kupplungskomponenten sind dem Fachmann aus der analytischen Chemie bekannte Verbindungen, die für photometrische Zwecke eingesetzt werden und die mit einer Reihe von Ionen z.B. stark gefärbte Verbindungen bilden. Beispiele hierfür sind 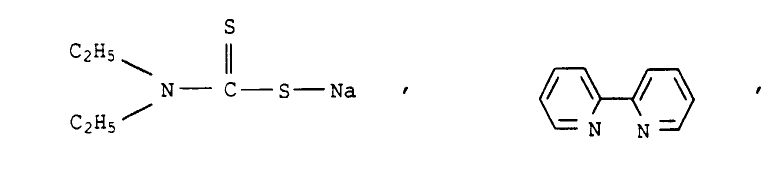 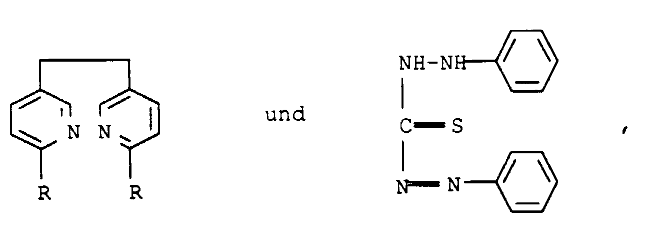 also

Dithiocarbamate, die z.B. mit Ionen von Fe, Co, Cd oder Hg reagieren, Cuproine oder Bipyridyle, die mit Cu-Ionen reagieren, Dithizone, die z.B. mit Pb-Ionen unter Bildung einer stark gefärbten Verbindung reagieren. Auch diese Verbindungen können so modifiziert werden, daß sie für die Langmuir-Blodgett-Technik geeignet sind oder in eine Mischschicht eingebracht werden können. Letzteres ist u.U. auch ohne eine spezielle Modifikation der Ausgangssubstanz möglich.

Aus der Wirts/Gast-Chemie (s. z.B. Vögtle, Supramolekulare Chemie, Teubner Verlag, Stuttgart (1990) ist bekannt, daß z.B. eine Reihe von Komplexen und Einschlußverbindungen zwischen kationischem oder neutralem Gastmolekül, z.B. Kronenmoleküle, Kryptanden, Cyclodextrine usw. mit Ionen eines Analyten entstehen, wobei es häufig zu starken Änderungen im Absorptionsspektrum kommen kann. Ein Beispiel hierfür ist die Reaktion
der Verbindung 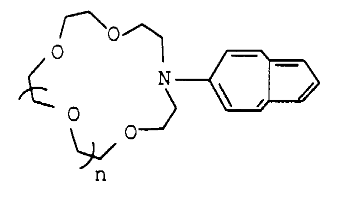 n = 1, 2
mit Ba-Ionen, wobei ein Farbumschlag von gelborange nach blauviolett erfolgt. Ein weiteres Beispiel wäre die Reaktion von 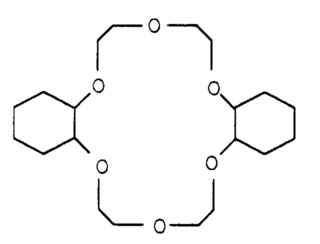 mit Sr-Ionen. Es ist auch die Einlagerung mehrerer Ionen in eine solche Käfigverbindung denkbar (s. F. Vögtle). Mit Hilfe der Wirts/Gast-Chemie lassen sich jedoch nicht nur z.B. Metallionen nachweisen, es ist sogar der Nachweis organischer Moleküle bis hin zu Biomolekülen möglich. So wird z.B. von S. Misumi, Pure Appl. Chem., 62(3) (1990) 493 beschrieben, daß bei der Reaktion von azobenzolhaltigen Kronenmolekülen 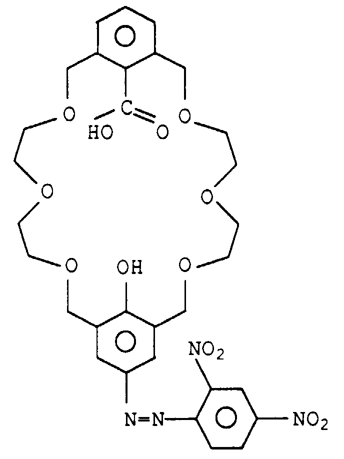 als Wirt
mit Aminen 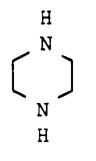 als Gast
eine deutliche Änderung des Absorptionsspektrums auftritt. Auch Cyclophane sind zum Nachweis organischer Moleküle geeignet (I.O. Sutherland, Pure Appl. Chem. 62(3) (1990) 499). So ist bis Zn-Porphyrin ein selektiver Wirt von 4,4-Bipyridyl 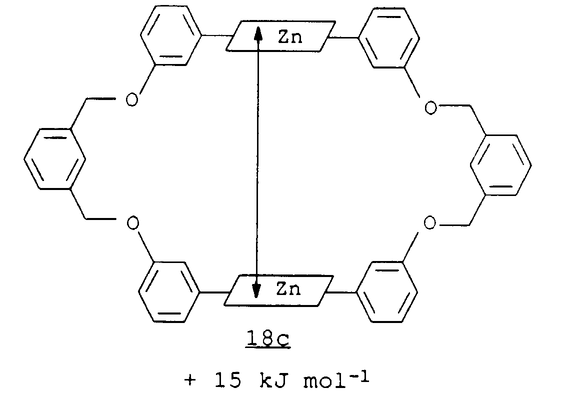 (Wirt) mit 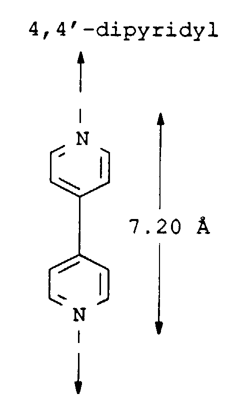 (Gast) Somit sind auch solche molekularen Systeme im Sinne der vorliegenden Erfindung als Akzeptorschicht nutzbar. Auf dieser Basis ist auch ein Nachweis von Biomolekülen oder Pharmakametaboliten denkbar.

Kronenether sind so modifizierbar, daß sie auch mit Hilfe der Langmuir-Blodgett-Technik übertragen werden können (s. S. Yoshida et al., Chem. Lett., Heft 2 (1989) 243); z.B. Oktadecyloxymethyl 18-Krone-6.

Die Selektivität des erfindungsgemäßen Sensors kann nicht nur durch geschickte Wahl der Donor- als auch der Akzeptorschicht beeinflußt werden; auch durch eine Vorbehandlung des Analyten durch pH-Einstellung und/oder Komplexierung kann die Selektivität erhöht werden, wie dem Fachmann aus photometrischen Nachweisreaktionen der analytischen Chemie bekannt ist.

Bei Verwendung geeigneter Kupplungskomponenten (K), z.B. unter Nutzung der Wirts/Gast-Chemie, ist es möglich, auch reversible Sensoren herzustellen. Die weiter oben beschriebene Nutzung photometrischer Reagenzien würde in der Regel nur zu "Einmalsensoren" führen, da die Kupplungsreaktion irreversibel ist.

Eine Reaktivierung der Sensorschicht, bzw. der Kupplungskomponente, ist durch Behandlung mit einer wäßrigen Lösung eines stärker komplexierenden Moleküls (z.B. EDTA) möglich, so daß der Sensor reversibel genutzt werden kann.

### Beispiel

Als Reagenzien Arachidinsäure (= C₂₀), Arachidinsäuremethylester (= AME), CuCl₂ und CdCl₂ wurden jeweils pa Materialien der Fa. Merck, Darmstadt eingesetzt.

Der Fluoreszenzfarbstoff 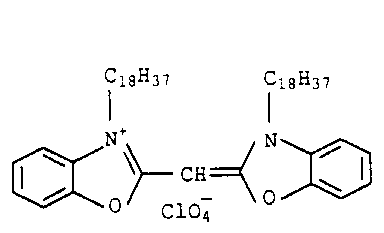 N',N-Dioctadecyl-oxacyaninperchlorat (S9)
wurde nach einer Vorschrift von Sondermann (Liebigs Ann. Chem. 749 (1971) 183) synthetisiert. Als Spreitungslösungsmittel wurde mit 1 Vol.-% Ethanol stabilisiertes Chloroform (Baker Chemicals, spectroscopic grade) verwendet. Dioctadecyldithiocarbamat 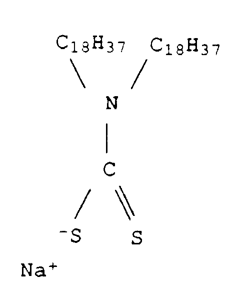 wurde entsprechend Th. Arndt et al., Thin Solid Films, 178 (1989) 319 synthetisiert und frisch vor dem Experiment in Chloroform gelöst. Als Subphase wurde entionisiertes und gereinigtes Wasser aus einem Milli-Q-System (Fa. Millipore) verwendet.

### Herstellung der Langmuir-Blodgett-Schichten

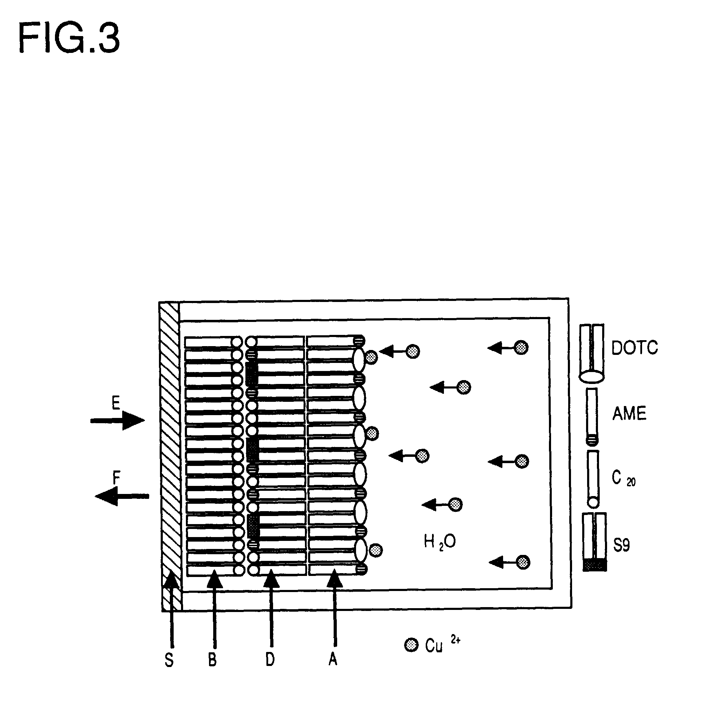

a) Donorschicht (D)
   Als festes Trägermaterial wurden Quarzplättchen
   (38 mm x 12 mm x 1 mm) nach Hydrophobisierung mit Dimethyldichlorsilan verwendet. Zunächst wurde eine Schicht Cadmiumarachidat bei 30 mN/m aufgebracht. Danach wurde bei gleichem Oberflächendruck eine Monoschicht übertragen, die auf Cadmium⁺²-Ionen enthaltender Subphase hergesellt wurde und den Fluoreszenzfarbstoff (S9) enthält (S9:AME:C₂₀=1:2:18).
b) Akzeptorschicht
   Die Schicht (A) wurde als 1:1 Mischung von Oktadecyldithiocarbamat (= DOTC) und AME bei 20 mN/m von einer wäßrigen Subphase auf eine Hälfte der mit Schicht (D) überzogenen Quarzplättchen übertragen, wobei DOTC im Sinne des erfindungsgemäßen Sensors die Kupplungskomponente (K) enthält. Die andere Hälfte des Substrates wurde als Referenz mit reinem AME beschichtet. Die beschichteten Substrate wurden unter Wasser aufbewahrt und dienten als Fenster einer kleinen Küvette (38 mm x 12 mm x 8 mm). Der Langmuir-Blodgett-Film steht so in Kontakt mit der wäßrigen Phase in der Küvette (s. Figur 3), die gegen die ionenhaltige Lösung ausgetauscht werden kann.
   Durch Reaktion mit dem zu untersuchenden Analyten, in diesem Fall einer Cu²⁺-Ionen haltigen Lösung, tritt eine Änderung des Absorptionsspektrums ein und die Schicht (A) wird im Sinne des Förster-Transfers zur Akzeptorschicht.

### Fluoreszenzspektroskopie und Nachweis des Sensoreffektes:

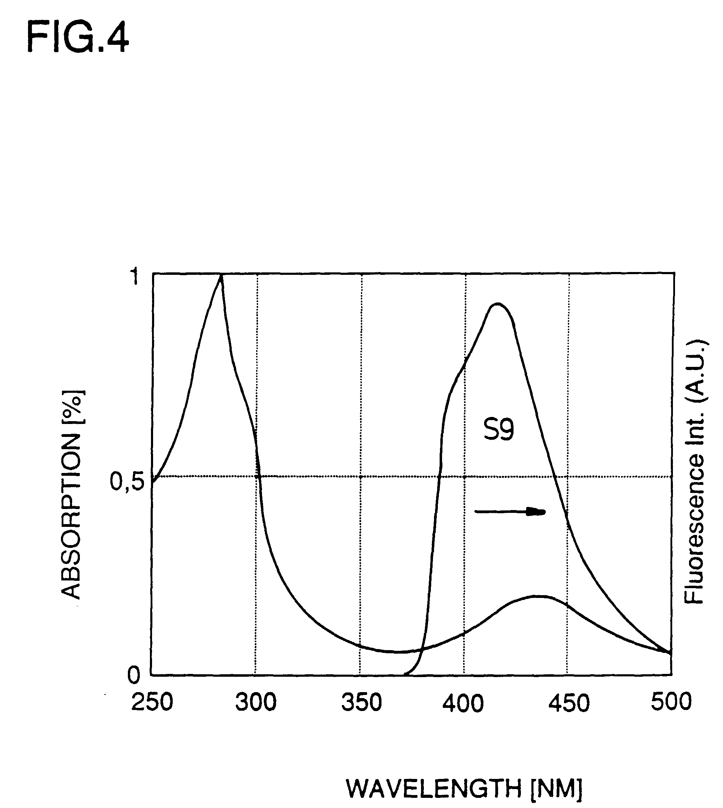

Für die Fluoreszenzmessungen wurde als Anregungsquelle eine Quecksilberdampflampe verwendet. Die Donormoleküle wurden bei 366 nm angeregt, die Emission bei 420 nm detektiert. Die Fluoreszenzintensität nimmt mit zunehmender Oberflächendichte der gebildeten DOTC-Kupfer-Komplexe ab. Bei einer Cu²⁺-Konzentration größer 10⁻²M beobachtet man ein ca. 70 %iges Quenchen der Anfangsfluoreszenz. Die Fluoreszenz des Donors ist in Figur 4 gezeigt. Eine Beeinflussung der Referenzhälfte wird nicht gefunden, d.h. ein Quenchen der Fluoreszenz durch das Kupferion allein findet nicht statt. Erst die Reaktion mit der Kupplungskomponente bewirkt den Sensoreffekt. Die Untersuchungen zeigen, daß der Sensor in der vorliegenden Konfiguration im Konzentrationsbereich zwischen 10⁻⁹ Mol und 10⁻² Mol arbeitet.

Im gewählten Beispiel beträgt der Abstand zwischen Donorfarbstoff und Akzeptor, der durch Wechselwirkung der Kupplungskomponente mit dem Analyten entsteht, etwa 5 nm, was grob dem kritischen Försterradius entspricht. Die Responsezeiten betragen weniger als 10 Sek. und sind primär durch die Küvettengeometrie limitiert.

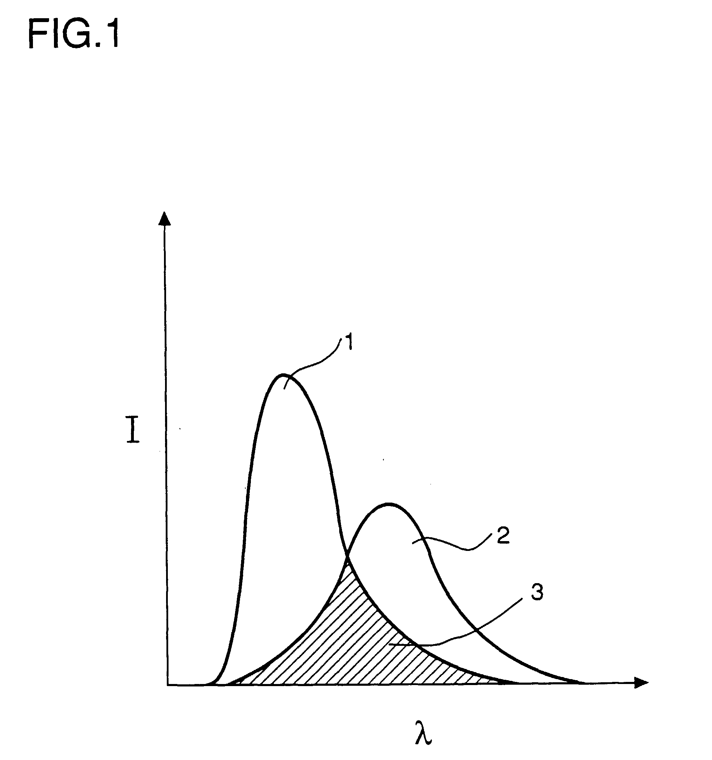

- Figur 1: veranschaulicht den Energie-Transfer, auf dem der Sensor basiert;
1 = Donor-Fluoreszenz;
2 = Akzeptor-Absorption;
3 = spektrale Überlappung.
- Figur 2: zeigt einen zur Aggregatbildung geeigneten Farbstoff mit den zugehörigen Spektren.
- Figur 3: zeigt den schematischen Aufbau eines Sensors; hierin bedeuten:
S = festes Trägermaterial,
B = Vorbehandlungsschicht aus Arachinsäure (C20),
D = Schicht (D), den Fluoreszenzfarbstoff (S9) enthaltend (S9 : AME : C20 =1:2:18),
A = Schicht (A), enthaltend die Kupplungskomponente (K),
E = Anregungslicht (366 nm),
F = Fluoreszenzlicht,
DOTC = Dioctadecyldithiocarbamat,
AME = Arachinsäuremethylester,
C₂₀ = Arachinsäure,
S9 = N',N-Dioctadecyl-oxacyaninperchlorat,
- Figur 4: zeigt die Überlappung des Absorptionsspektrums des DOTC-Cu-Komplexes mit dem Fluoreszenzspektrum des Donor-Farbstoffes S9.

## Patentansprüche

1. Chemischer Sensor, bestehend aus
(a) einem festen Trägermaterial,
(b) einer auf dem Trägermaterial aufgebrachten Schicht (D), die mindestens eine fluoreszierende Verbindung enthält, und
(c) einer auf der Schicht (D) aufgebrachten Schicht (A), die eine oder mehrere Kupplungskomponenten (K) enthält, die durch Reaktion mit dem Analyten eine oder mehrere spezifische Absorptionsbanden hervorrufen, die mit der Emission der fluoreszierenden Verbindung der Schicht (D) teilweise oder vollständig spektral überlappen.

2. Chemischer Sensor nach Anspruch 1, dadurch gekennzeichnet, daß zwischen Schicht (D) und Schicht (A) eine Zwischenschicht (Z) mit definiertem Dipolmoment zur Kontrolle der chemischen Gleichgewichte in den benachbarten Schichten angebracht ist.

3. Chemischer Sensor nach einem der vorhergehenden Ansprüche, dadurch gekennzeichnet, daß die auf dem festen Trägermaterial aufgebrachten Schichten insgesamt eine Dicke zwischen 20 und 200 Å aufweisen.

4. Chemischer Sensor nach einem der vorhergehenden Ansprüche, dadurch gekennzeichnet, daß das feste Trägermaterial aus Quarz oder Glas besteht und gegebenenfalls vorbehandelt ist.

5. Chemischer Sensor nach einem der vorhergehenden Ansprüche, dadurch gekennzeichnet, daß die Kupplungskomponenten (K) der Schicht (A) an der Oberfläche des Sensors in direktem Kontakt mit der Lösung des Analyten sind.

6. Chemischer Sensor nach einem der vorhergehenden Ansprüche, dadurch gekennzeichnet, daß der Abstand zwischen fluoreszierender Verbindung der Schicht (D) und der Kupplungskomponente der Schicht (A) dem Försterradius entspricht.

7. Chemischer Sensor nach einem der vorhergehenden Ansprüche, dadurch gekennzeichnet, daß die fluoreszierende Verbindung der Schicht (D) in einer Polymer- oder Monomermatrix verteilt und/oder gebunden vorliegt.

8. Chemischer Sensor nach einem der vorhergehenden Ansprüche, dadurch gekennzeichnet, daß in der Schicht (D) J- oder Scheibe-Aggregate ausgebildet sind.

9. Chemischer Sensor nach einem der vorhergehenden Ansprüche, dadurch gekennzeichnet, daß die Kupplungskomponente (K) der Schicht (A) eine Verbindung ist, die mit Ionen oder organischen Molekülen im Sinne einer Wirts/Gast-Reaktion wechselzuwirken vermag.

10. Chemischer Sensor nach einem der vorhergehenden Ansprüche, dadurch gekennzeichnet, daß die Kupplungskomponente (K) der Schicht (A) aus einem oder mehreren Komplexbildnern besteht, der mit Ionen reagiert.

11. Chemischer Sensor nach einem der vorhergehenden Ansprüche, dadurch gekennzeichnet, daß die Schichten (D) und (A) sowie gegebenenfalls (Z) nach der Laugmuir-Blodgett und/oder Lagmuir-Schäfer-Technik erhalten worden sind.

## Claims

1. A chemical sensor consisting of
(a) a solid substrate,
(b) a layer (D) which is applied to the substrate and contains one or more fluorescent compounds and
(c) a layer (A) which is applied to the layer (D) and contains one or more coupling components (K) which, as a result of reacting with the analyte, give rise to one or more specific absorption bands, some or all of which overlap in the spectrum with the emission of the fluorescent compound of layer (D).

2. A chemical sensor as claimed in claim 1, wherein an intermediate layer (Z) having a defined dipole moment is applied between layer (D) and layer (A) to control the chemical equilibria in the adjacent layers.

3. A chemical sensor as claimed in any of the preceding claims, wherein the layers applied to the solid substrate have a total thickness of from 20 to 200 Å.

4. A chemical sensor as claimed in any of the preceding claims, wherein the solid substrate consists of quartz or glass and may be pretreated.

5. A chemical sensor as claimed in any of the preceding claims, wherein the coupling components (K) of layer (A) on the surface of the sensor are in direct contact with the solution of the analyte.

6. A chemical sensor as claimed in any of the preceding claims, wherein the distance between the fluorescent compound of layer (D) and the coupling component of layer (A) corresponds to the Förster radius.

7. A chemical sensor as claimed in any of the preceding claims, wherein the fluorescent compound of layer (D) is present, distributed and/or bound in a polymer or monomer matrix.

8. A chemical sensor as claimed in any of the preceding claims, wherein J or disk aggregates are formed in layer (D).

9. A chemical sensor as claimed in any of the preceding claims, wherein the coupling component (K) of layer (A) is a compound which is capable of interacting with ions or organic molecules by a host/guest reaction.

10. A chemical sensor as claimed in any of the preceding claims, wherein the coupling component (K) of layer (A) consists of one or more complexing agents which react with ions.

11. A chemical sensor as claimed in any of the preceding claims, wherein layers (D) and (A) and, where relevant, (Z) have been obtained by the Langmuir-Blodgett and/or Langmuir-Schäfer method.

## Revendications

1. Détecteur chimique, consistant en
(a) un matériau support solide,
(b) une couche (D) disposée sur le matériau support, qui contient au moins un composé fluorescent, et
(c) une couche (A) disposée sur la couche (D), qui contient un ou plusieurs composants de couplage (K), qui provoquent par réaction avec l'analyte une ou plusieurs bandes d'absorption spécifiques, qui se chevauchent spectralement partiellement ou totalement avec l'émission du composé fluorescent de la couche (D).

2. Détecteur chimique selon la revendication 1, caractérisé par le fait que, entre la couche (D) et la couche (A) est disposée une couche intermédiaire (Z) ayant un moment dipolaire défini pour le contrôle des équilibres chimiques dans les couches voisines.

3. Détecteur chimique selon l'une des revendications précédentes, caractérisé par le fait que les couches disposées sur le matériau support solide présentent au total une épaisseur comprise entre 20 et 200 Å.

4. Détecteur chimique selon l'une quelconque des revendications précédentes, caractérisé par le fait que le matériau support solide consiste en du quartz ou du verre et est éventuellement prétraité.

5. Détecteur chimique selon l'une quelconque des revendications précédentes, caractérisé par le fait que les composants de couplage (K) de la couche (A) sont sur la surface du détecteur en contact direct avec la solution d'analyte.

6. Détecteur chimique selon l'une quelconque des revendications précédentes, caractérisé par le fait que la distance entre le composé fluorescent de la couche (D) et le composant de couplage de la couche (A) correspond au rayon de Förster.

7. Détecteur chimique selon l'une quelconque des revendications précédentes, caractérisé par le fait que le composé fluorescent de la couche (D) est réparti dans une matrice de polymère ou de monomère et/ou est présent à l'état lié.

8. Détecteur chimique selon l'une quelconque des revendications précédentes, caractérisé par le fait que dans la couche (D) son formés des agrégats en J ou en rondelle.

9. Détecteur chimique selon l'une quelconque des revendications précédentes, caractérisé par le fait que le composant de couplage (K) de la couche (A) est un composé qui est susceptible d'interaction avec des ions ou des molécules organiques au sens d'une réaction molécule incluante/molécule d'inclusion.

10. Détecteur chimique selon l'une quelconque des revendications précédentes, caractérisé par le fait que le composant de couplage (K) de la couche (A) consiste en un ou plusieurs complexants, qui réagit avec des ions.

11. Détecteur chimique selon l'une quelconque des revendications précédentes, caractérisé par le fait que les couches (D) et (A), ainsi qu'éventuellement (Z), ont été obtenues selon la technique de Langmuir-Blodgett et/ou de Langmuir-Schäfer.
